# EUROPEAN PATENT SPECIFICATION

(11) **EP 1 347 744 B1**
(45) Date of publication and mention of the grant of the patent: **08.09.2004**
(21) Application number: 01995695.2
(22) Date of filing: 18.12.2001
(51) Int. Cl.: A61K 9/14

(54) **SOLID DISPERSIONS OF NITRATE ACTIVE PRINCIPLES**
FESTE DISPERSIONEN MIT NITRATAKTIVEN WIRKSTOFFEN
DISPERSIONS SOLIDES DE PRINCIPES ACTIFS DE NITRATE

(30) Priority: 22.12.2000 IT MI20002803
(43) Date of publication of application: 01.10.2003
(60) Divisional application: 04103272.3
(73) Proprietor: Nicox S.A., 06906 Sophia Antipolis Cedex (FR)
(72) Inventor: TRESPIDI, Laura, I-26026 Pizzighettone (IT); DEL SOLDATO, Piero, I-20052 Monza (IT)
(74) Representative: Barchielli, Giovanna
(86) International application number: PCT/EP2001/014967
(87) International publication number: WO 2002/051385

(56) References cited:
- WO-A-01/15677
- MINGHETTI, P.; ET AL.: "Application of solubility parameter in nitroflurbiprofen topical semisolid formulations" PROCEEDINGS OF THE INTERNATIONAL SYMPOSIUM ON CONTROLLED RELEASE OF BIOACTIVE MATERIALS 27TH, 7 - 13 July 2000, pages 936-937, XP002199487 Paris (FR)

## Description

### FIELD OF THE INVENTION

The present invention relates to solid dispersions of nitrate active principles characterized by an increased dissolution rate and/or apparent solubility of said active principles and to a method for their production.

### STATE OF THE ART

The applicant has developed a number of active principles, characterized by the presence in their structure of a nitro group, having remarkably advantageous pharmacological properties. These active principles are described in the patents: EP670825, EP722434, EP759899, EP609415, US5703073, and in the patent applications WO98/15568, WO98/21193, WO00/51988, WO00/61537, WO00/61541, WO00/61604, WO00/25776, MI99A001817.

Unfortunately, the utility of many of the above mentioned active ingredients is limited by their scarce solubility in water, which results in an insufficient and irregular absorption and a slow onset of the pharmacological action. This last aspect is particularly problematic in case of active ingredients such as, for instance, antinflammatory active ingredients and/or analgesics for which a rapid onset of the therapeutic action is of fundamental importance.

Thus, there is as need to develop new pharmaceutical formulations for the administration of nitrate active principles which, compared with traditional formulations, are characterized by an improved bioavailability and a faster onset of action. It is known that the dissolution rate of poor water soluble drugs can be increased by their conversion to the corresponding amorphous forms. A technique which can be used to this purpose is the formation of a solid dispersion of the active agent in an inert matrix, usually of polymeric nature. Nevertheless, this technique does not always allow to obtain the amorphous form and consequently the increase in dissolution rate of the active agent. Several parameters such as, for instance the interactions between the polymer and the active ingredient, the ratio between then and the production technique adopted influence the chemical-physical features of the solid dispersion obtained. Thus, for each particular active ingredient it is necessary to select both the polymer and the operative conditions for the preparation of the dispersion that lead to the conversion to the amorphous form.

### SUMMARY OF THE INVENTION

The inventors have now found that it is possible to obtain an increase in the dissolution rate and/or the apparent solubility and consequently in the bioavailability of nitrate active principles by forming solid dispersions of said active principles characterized in that the inert matrix includes at least one polymer chosen among polyvinyl pyrrolidone, cellulose ethers and polyethylene glycols.

Therefore, the present invention refers to solid dispersions comprising at least one nitrate active principle and a hydrophilic polymer chosen among polyvinyl pyrrolidone, cellulose ethers and polyethylene glycols.

### DESCRIPTION OF THE FIGURES

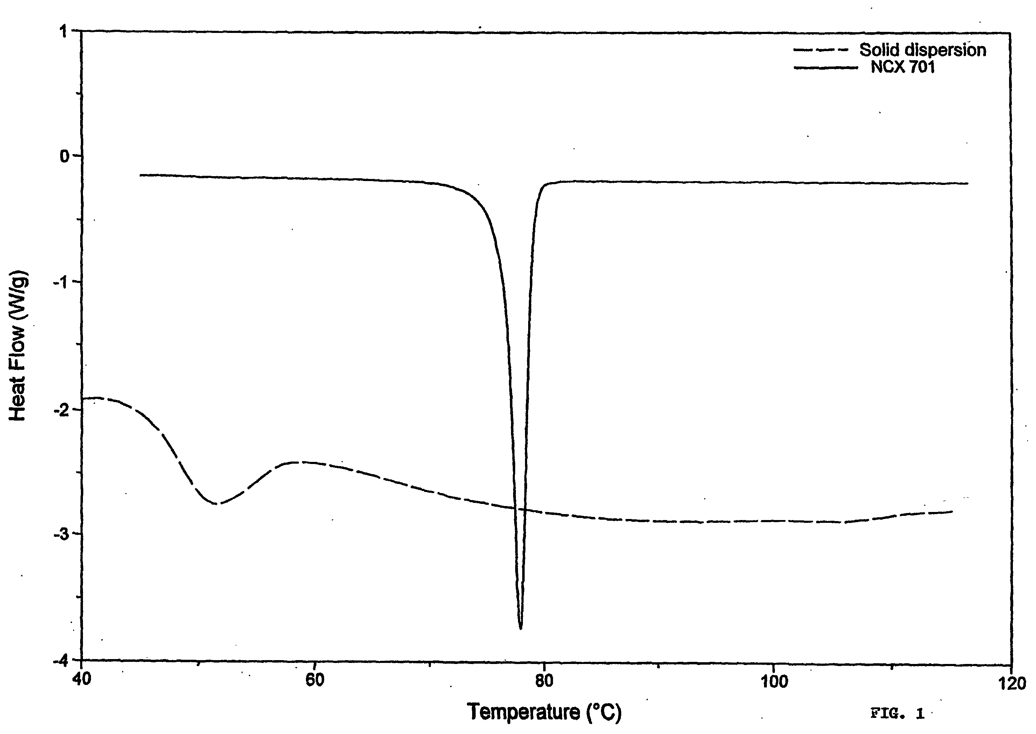

Figures 1, 2 and 3 show the thermograms of the crystalline form and of the amorphous solid dispersion according to the present invention of the following derivatives:
   4- acetylaminophenyl ester of 4 nitroxybutanoic acid (NCX701)
   2- (acetyloxy-benzoic-acid-3-nitroxymethyl) phenyl ester (NCX 4016)
   (hydroxycortisone 21-[(4'nitroxymethyl)benzoate) (NCX 1022)

### DETAILED DESCRIPTION OF THE INVENTION

The present invention relates to solid dispersions comprising at least one nitrate active ingredient in amorphous form and a hydrophilic polymer chosen among polyvinyl pyrrolidone, preferably having a molecular weight comprised between that of the polyvinyl pyrrolidone K17 and that of polyvinifpyrrolidone K30, cellulose ethers and polyethylene glycol, preferably having a molecular weight higher than that of PEG 1000, and more preferably PEG with a molecular weight higher than that of PEG 1500 and lower than that of PEG 6000. Among the cellulose ethers particularly preferred is the hydroxypropylmethylcellulose, preferably having a viscosity at 20°C, in a 2% aqueous solution, lower than 50 cPs, and preferably hydroxypropylmethylcellulose with viscosity comprised between 5 and 50 cPs.

By "nitrate active principles" it is meant compounds having formula (I).

A-X₁-L-(W)ₚNO_{q} (I)

wherein:
**p** is an integer equal to 1 or 0;
**q** is an integer equal to 1 or 2;
**A=R-T**_{**1**}**-**, wherein **R** is the radical of a pro-drug having formula **R-T**_{**1**}**-Z**, chosen among the therapeutic classes of drugs reported here after, wherein
**Z** is **H, OH, NH**_{**2**}**, NHR**_{**3**}**, N(R**_{**3**}**)**_{**2**}, wherein **R**_{**3**} is a linear or branched C₁-C₅ alkyl radical
**T**_{**1**} **= (CO)**_{**t**} **or (X)**_{**t**}**,** wherein X = an oxygen atom, a sulphur atom or NR₂ wherein
**R**_{**2**} is hydrogen or a linear or branched alkyl, having from 1 to 5 carbon atoms, t and t' are integer and equal to zero or 1, provided that t = 1 when t' = 0; t = 0 when t'=1;
**X**_{**1**} **= -T**_{**B**}**-Y-T**_{**BI**} wherein T_{B} and T_{BI} are the same or different
**T**_{**B**} **= (CO)** when **t** = 0, **T**_{**B**} = **X** when t' = 0, being X as above defined;
**T**_{**BI**} **= (CO)**_{**tx**} or **(X)**_{**txx**} wherein tx and txx are 0 or 1; with the proviso that tx = 1 when txx = 0; tx = 0 when txx = 1; X is as above defined;
**Y** is a bivalent bridging group chosen among the following:
   **1)** 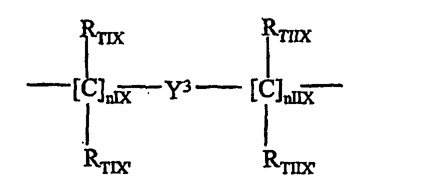 wherein:
      **nIX** is an integer comprised between 0 and 3, preferably 1;
      **nIIX** is an integer comprised between 1 and 3, preferably 1;
      **R**_{**TIX**}**, R**_{**TIX**}**, R**_{**TIIX**}**, R**_{**TIIX**^{**'**}}, equal or different from one another, are H or linear or branched C₁-C₄ alkyl; preferably R_{TIX}, R_{TIX}, R_{TIIX}, R_{TIIX'} are H.
      **Y**^{**3**} is a saturated , unsaturated or aromatic heterocyclic ring having 5 or 6 atoms and containing one or two nitrogen atoms, Y³ is preferably chosen among the following bivalent radical: 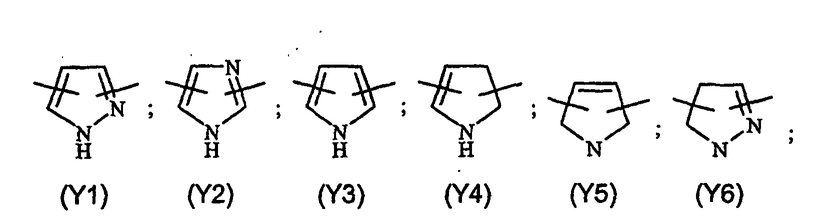 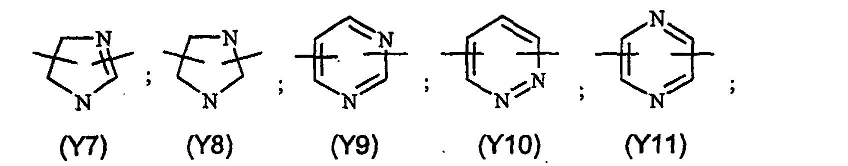 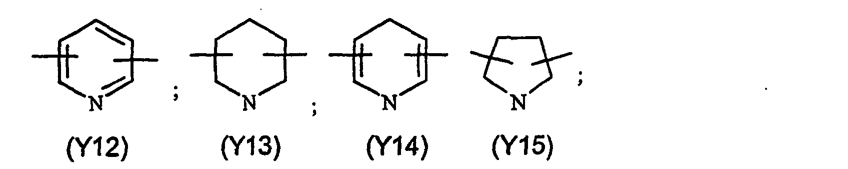 wherein (Y12) is preferred;
   **2)** an alkylene group R' wherein R' is C₁-C₂₀ linear or branched when possible, having preferably 2 to 6 carbon atoms, optionally substituted with at least one of the following groups: -NH₂, -OH or -NHCOR₃, wherein R₃ is a linear or branched C₁₋₅ alkyl;
   **3)** a cycloalkylene having from 5 to 7 carbon atoms, optionally substituted with side chains R', wherein R' is as defined above, and at least one carbon atom of the cycloalkylenic ring can be optionally substituted with etheroatoms.
   **4)** 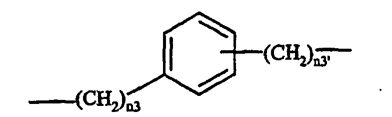 wherein n3 is an integer from 0 to 3 and n3' is an integer from 1 to 3;
   **5)** 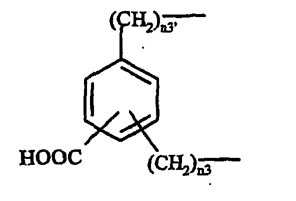 wherein n3 and n3' have the above indicated meaning,
   **6)** 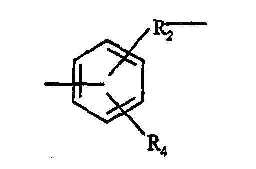 wherein
      R₄ is hydroxy, hydrogen, alkoxy R₅O- wherein R₅ is a linear, branched or cyclic C₁₋₁₀ alkyl group, preferably R₅ is a methyl group;
      R₂ is a linear or branched C₂₋C₁₀ alkenyl group, including at least one double bond, preferably R₂ is the ethenylene group (-CH=CH-);
   **7)** 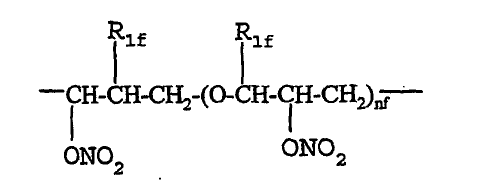 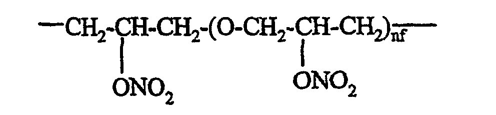 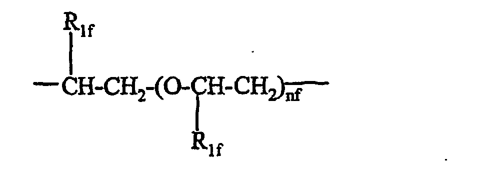 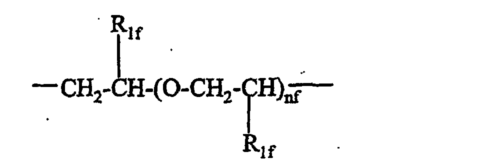 wherein R_{1f} = H, CH₃ and nf is an integer from 0 to 6; preferably froml to 4;
   **8)** or Y is the bivalent radical whose precursor Z-T_{B}-Y-T_{Bl}-Z, wherein Z is as defined above and it is chosen among the following compounds:
      aspartic acid, histidine, 5-hydroxytryptophan, 2-thiouracil, 2-mercaptoethanol, hesperidine, secalcipherol, 1-α-OH-Vitamin D2, flocalcitriol, 22-oxacalcitriol, 24,28-methylen-1α-hydroxyvitamin D2, succinic acid, L-carnosine, anserine, selenocysteine, selenomethionine, penicillamine, N-acetylpenicillamine, cysteine, N-acetylcysteine, glutathione, gallic acid, ferulic acid, gentisic acid, citric acid, caffeic acid, hydrocaffeic acid, p-coumaric acid, vanillic acid, chlorogenic acid, kynureic acid, siringic acid, nordihydroguaiaretic acid, quercetin, catechin, kaempferol, sulfuretin, ascorbic acid, isoascorbic acid, hydroquinone, gossypol, reductic acid, methoxyhydroquinone, hydrohydroxyquinone, propyl gallate, saccharose, 3,5-di-ter-butyl-4-hydroxybenzyl-thioglycolate, allopurinol, conyferyl alcohol, 4-hydroxyphenethyl alcohol, p-coumaric alcohol, curcumin, N,N'-diphenyl-p-phenylenediamine, thionine, hydroxyurea, 3,3'-thiodipronic acid, fumaric acid, di-hydroxymaleic acid, N-methylendiethanolamine, thiodiethylenglycol, 1,4-dioxane-2,6-dimethane, tetrahydropyran-2,6-di-methanol, 4H-pyran-2,6-di-methanol, cyclohexene-1,5-dimethanol, 1,4-dithian-2,fi-dimethanol, thiophene-2,5-di-methanol, oxazole-2,5-di-methanol.

      **L**= covalent bond, or **L = X, X** being as defined above, **L = (CO)**
      **W = Y**_{**T**}**-X-** wherein **Y**_{**T**} has the same meanings of **Y**, but is different from **Y**,
      **R-T**_{**1**}**-Z** is chosen among the following drugs:
      - Non steroidal anti-inflammatory drugs: aceclofenac, acemetacin, acetylsalicylic acid, alclofenac, alminoprofen, amfenac, ampiroxicam, balsalazide, bendazac, bermoprofen, α-bisabolol, bromfenac, bromosaligenin, bucloxic acid, butibufen, carprofen, cinmetacin, clidanac, clopirac, diclofenac, CS-670, diflunisal, ditazol, enfenamic acid, etodolac, etofenamate, felbinac, fenbufen, fenclozic acid, fendosal, fenoprofen, fentiazac, fepradinol, flufenamic acid, flunixin, flunoxaprofen, flurbiprofen, giucametacin, glycol salicylate, ibuprofen, ibuproxam, indomethacin, indoprofen, isofezolac, isoxepac, isoxicam, ketoprofen, ketorolac, lomoxicam, loxoprofen, mechlofenamic acid, mefenamic acid, meloxicam, mesalamine, metiazinic acid, mofezolac, naproxen, niflumic acid, olsalazine, oxaceprol, oxaprozin, oxifenbutazone, parsalmide, pemedolac, perisoxal, phenyl acetylsalicilate, pirazolac, piroxicam, pirprofen, pranoprofen, protizinic acid, salacetamide, salicylamido-O-acetic acid, saliciylsulforic acid, salsalate, sulindac, suprofen, suxibuzone, tenidap, tenoxicam, thiaprofenic acid, thiaramide, tinoridine, tolfenamic acid, tolmetin, tropesin, xenbucin, ximoprofen, zaltoprofen, zomepirac, tomoxiprol,
         Analgesics: paracetamol, acetaminosalol, aminochlorthenoxazin, acetylsalicitic acid, 2-amino-4-picoline, acetylsalicylsalicilyc acid, anileridine, benoxaprofen, benzylmorphine, 5-bromosaliciylic acid acetate, bucetin, buprenorfine, butorfanol, capsaicin, cincofenol, ciramadol, clometacine, clonixin, codeine, desomorphine, dezocine, dihydrocodeine, dihydromorphine, dimefeptanol, dipyrocetyl, eptazocine, etoxazen, ethylmorphine, eugenol, floctafenine, fosfosal, glafenine, hydrocodon, hydromorone, hydroxypetidine, ibufenac, p-lactophenetide; levorfanol, meptazinol, metazocine, metopon, morphine, nalbuphine, nicomorphine, norlevorfanol, normorphine, oxycodone, oxymorphon, pentazocine, fenazocine, fenocoll, fenoperidine, fenilbutazone, phenylsalicylate, phenilramidol, salicin, salicylamide, tiorphan, tramadol, diacereine, actarit;
      - Steroids: chenodeoxycholic acid, ursodeoxycholic acid, alclomethasone, algestone, amcinonide, beclomethasone, betamethasone, budesonide, chlorprednisone, clobetasol, clobethasone, clocortolone, cloprednol, corticosteron, cortisone, cortivazol, deflazacort, desonide, desoximethasone, dexamethasone, diflorasone, diflucortolone, difluprednate, estradiol, ethynilestradiol, fluazacort, flucloronide, flucortyn butyl, flumethasone, flunisolide, fluocinolone acetonide, fluocinonide, fluocortolone, fluorometholone, fluperolone acetate, fluprednidene acetate, fluprednisolone, flurandrenolide, fluticasone propionate, formocortal, halcinonide, halobetasol propionate, halomethasone, halopredone acetate, hydrocortamate, hydrocortisone, loteprednol etabonate, mazipredone, medrysone, meprednisone, mestranot, metilprednisotone, mitatrienediol, mometasone furcate, moxestrol, paramethasone, prednicarbate, prednisolone, prednisolone 25-diethylaminoacetate, prednisone, prednival, prednylidene, rimexotone, 21-acetoxy-pregnenolone, triamcinolone hexacetonide, triamcinolone acetonide, triamcinolone, tixocortol;
      - Bronchodilatory drugs: acephilline, albuterol, bambuterol, bamiphylline, bevonium methyl sulfate, bitolterol, carbuterol, clenbuterol, clorprenaline, dioxetedrine, diphylline, ephedrine, epinephrine, eprozinol, etaphedrine, ethylnorepinephrine, etophylline, fenoterol, flutoprium bromide, hexoprenaline, ipratropium . bromide, isoetarine, isoprotenerol, mabuterol, metaprotenerol, oxitropium bromide, pirbuterol, procaterol, protokylol, proxyphylline, reproterol, rimiterol, salmeterol, soterenol, terbutatine, 1-theobromoacetic acid, thiotropium bromide, tretoquinolol, tulobuterol, oxybutinyn, zaprinast.
      - Expectorants and mucolitic agents: ambroxol, bromexine, domiodol, erdosteine, guaiacol, guaifenesine, glycerol iodurate, letosteine, mesna, sobrerol, stepronin, terpin, thiopronin;
      - Anti-asthmatic, antiallergic and antihistaminic drugs: acrivastine, alloclamide, amlexanox, cetirizine, clobenzepam, chromoglycate, chromolyn, epinastine, fexofenadine, formoterol, hystamine, hydroxyzine, levocabastine, lodoxamide, mabuterol, metron s, montelukast, nedocromil, repirinast, seratrodast, suplatast tosylate, terfenadine, tiaramide, bromexine, formoterol;
      - ACE-inhibitors: alacepril, benazepril, captopril, ceronapril, cilazapril, delapril, enalapril, enalaprilat, fosinopril, imidapril, lisinopril, losartan, moveltipril, naftopidil, perindopril, quinapril, ramipril, spirapril, temocapril, trandolapril, urapidil;
      - β-blockers: acebutolol, alprenolol, amosulalol, arotinolol, atenolol, betaxolol, bevantolol, bucumolol, bufetolol, bufuralol, bunitrolol, bupranolol, butofilol, carazolol, carteolol, carvedilol, celiprolol, cetamolol, ditevatol, epanolol, esmolol, indenolol, labetalol, mepindolol, metipranolol, metoprolol, moprolol, nadotol, nadoxolol, nebivolol, nifenalol, nipridalol, oxprenolol, penbutolol, pindolol, practolol, pronetalol, propranolol, sotalolol, suffinalolol, talinolol, tertatolol, tilisolol, timolol, toliprolol, xibenolol;
      - Drugs for vascular disorders: acetorphan, acetylsalicylic acid, argatroban, bamethan, benfurodil hemisuccinate, benziodarone, betaistine, brovincamine, bufeniode, citicoline, clobenfurol, clopidogrel, cyclandelate, heparine, datteparin, dipiradamol, droprenilamine, enoxaparin, fendiline, ifenprodil, iloprost, indobufen, isbogrel, isoxsuprine, lamifiban, nadroparin, nicotinoyl alcohol, nylidrin, ozagrel, perhexiline, prenilamine, papaveroline, reviparin sodium salt, ridogrel, suloctidil, tinophedrine, tinzaparin, triflusal, xanthinol niacinate, fenilpropanolamine, midodrine;
      - Antidiabetics: acarbose, carbutamide, glibomuride glybuthiazol, miglitol, repaglinide, troglitazone, 1-buthyl-3-methanyl-urea, tolrestat, nicotinamide;
      - Antitumoral drugs: ancitabine, anthramicine, azacitidine, azaserine, 6-azauridine, bicalutamide, carubicine, carzinophilin, chlorambucil, chlorozotocin, citarabine, daunorubicine, defosfamide, demecolcine, denopterine, 6-diazo-5-oxo-L-norleucine, docetaxel, doxifluridine, doxorubicine, droloxifene, edatrexate, eflornithine, enocitabine, epirubicine, epitiostanol, etanidazole, etoposide, fenretinide, fludarabine, fluorouracyl, gemcitabine,. hexestrol, idarubicine, lonidamine, mannomustine, melphalan, menogaril, 6-mercaptopurine, methotrexate, mitobronitol, mitolactol, mitomycins, mitoxantrone, mopidamol, micophenolic acid, ninopterine, nogalamycin, paclitaxel, pentostatin, pirarubicin, piritrexim, plicamicine, podofillic acid, porfimer sodium, porfiromycin, propagermanium, puromycin, ranimustine, retinoic acid, roquinimex, streptonigrin, streptozocin, teniposide, tenuazonic acid, tiamiprine, thioguanine, tomudex, topotecan, trimetrexate, tubercidin, ubenimex, vinblastine, vincristine, vindesine, vinorelbine, zorubicine;
      - Antiulcer drugs: ε-acetamidocaproic acid, arbaprostil, cetraxate, cimetidine, ecabet, enprostil, esaprazole, irsogladine, misoprostol, omeprazot, omoprostil, pantoprazol, plaunotol, rioprostil, rosaprostol, rotraxate, sofalcone, trimoprostil;
      - Antihyperlipidemic drugs: atorvastatine, cilastatine, dermostatine, fluvastatine, lovastatine, mevastatine, nistatine, pentostatine, pepstatine, privastatine sodium salt, simvastatine;
      - Antibacterial drugs: amdinocillin, amoxicillin, ampicillin, apalcillin, apicyclin, aspoxicillin, azidamfenicol, azidocillin, azlocillin, aztreonam, benzoylpas, benzyl penicillinic acid, biapenem, bicozamycin, capreomycin, carbenicillin, carindacillin, carumonam, cefaclor, cefadroxil, cefamandole, cefatrizine, cefazedone, cefazoline, cefbuperazone, cefclidin, cefdinir, cefditoren, cefepime, cefetamet, cefixime, cefmenoxime, cefmetazole, cefminox, cefodizime, cefonicid, cefoperazone, ceforanide, cefotaxime, cefotetan, cefotiam, cefoxitin, cefozopran, cefpimizole, cefpiramide, cefpirome, cefprozil, cefroxadine, cefsulodin, ceftazidime, cefteram, ceftezole, ceftibuten, ceftiofur, ceftizoxime, ceftriaxone, cefuroxime; cefuzonam, cephacetrile sodium, cephalexin, cephaloglycin, cephaloridine, cephalosporin C, cephalothin, cephapirin sodium, cephradine, chloramphenicol, chlortetracicline, cinoxacine, clavulanic acid, clofoctol, clometocilline, cloxacilline, cyclacilline, cycloserine, demeclocicline, dicloxacillin, epicillin, fenbecillin, flomoxef, floxacillin, hetacillin, imipenem, lenampicillin, loracarbef, lymecycline, mafenide, meclocycline, meropenem, metampicillin, metacicline, meticillin sodium salt mezlocillin, minocicline, moxalactam, mupirocin, myxin, negamycine, novobiocin, oxacillin, pamipenem, penicillin G potassium salt, penicillin N, penicillin O, penicillin V, pheneticillin potassium salt, pipaciclyne, piperacillin, pirlimycin, porfiromycin, propicillin, quinacillin, ritipenem, rolitetracycline, sancycline, sedecamycin, spectinomycin, sulbactam, sulbenicillin, temocillin, tetracycline, ticarcillin, tigemonam, tubercidine, argininsa, arbekacin, apramycin, amikacin, azithromycin, bacampicillin, cefcapene pivoxil, cefpodoxime proxetil dapsone, deoxydihydrostreptomycin dibekacin, etambutol, flumequine, guamecycline, nifurpirinol, nifurprazine, nitroxoline, glyconiazide, isoniazide; opiniazide, mupirocin, negamydn, netilmicyn, pipacycline, fortimycins, gentamycin, ibostamycin, lincomycin, micronomycin, midecamycin, miokamycin, oleandomycin, paromomycin, rosaramycin, sisomycin, streptomycin, tobramycin, trospectomycin, claritromycin, diritromycin, enviomycin, erithromyan, josamycin, midecamycin, miocamycin, rifabutine, rifamide, rifamycin, rifaxymine, rokitamycin, spiramycin, troleandromycin, viomycin, virginiamycin; p-aminosalicylic acid, benzilpenicillinic acid, acetil sulfametossipirazine, acediasulfone, 4-sulfanylamidosalicylic acid, 4,4'-sulfinyldianiline, 4'-(methylsulfamoyl)sulfanylanilide, 2-p-sulfanilylanilinoethanol, N-sulfanilyl-3,4-xylamide, p-sulfanilylanilinoethanol, p-sulfanilylbenzylamine, salazosulfadimidine, salinazid, succisulfone, sulfabenzamide, sulfacetamide, sulfachlorpiridazine, suffachrysoidine, sulfacitine, sulfadiazine, sulfadicramide, sulfadimetoxine, sulfadoxine, sulfaetidol, sulfaguanidine, sulfaguanole, sulfalene, sulfamerazine, sulfameter, sulfametazine, sulfametizol, sulfamethomidine, sulfametoxazol, sulfametoxypiridazine, sulfamethyltiazol, sulfametrole, suffamidochrysoidine, sulfamoxole, sulfanylamide, sulfanylilurea, sulfaperine, sulfafenazol, sulfaproxyline, sulfapyrazine, sulfapyridine, sulfasomizole, sulfasymazine, sulfatiazol, sulfathiourea, sulfisomidine, sulfisoxazol, sultamicillin, tiazosulfone, mafenide, clofazimine, carbomycin, clomocycline, meclocycline, metampicillin, meticillin, metronidazole, mezlocillin, moxalactam, oxytetracycline, piromidic acid, pivampicillin, ciprofloxacin, clinafloxacin, difloxacin, enoxacin, enrofloxacin, fleroxacin, grepafloxacin, lomefloxacin, norfloxacin, ofloxacin, pazufloxacin, pefloxacin, rifanpin, rufloxacin, talampicillin, trovafloxacin, tosufloxacin, sparfloxacin;
      - Antiviral drugs: aciclovir, amantadine, cidofovir, cytarabine, didanosine, dideoxyadenosine, edoxudine, famciclovir, floxuridine, ganciclovir, idoxuridine, indanavir, kethoxal, lamivudine, MADU, penciclovir, podophyflotoxine, ribavirine, rimantadine, saquinavir, sorivudine, stavudine, trifluridine, valacyclovir, vidarabine, xenazoic acid, zaicitabine, zidovudine;
      - Inhibitors of bone reabsorption: aleridronic acid, butedronic acid, etidronic acid, oxydronic acid, pamidronic acid, risedronic acid;
      - Drugs for dementia: amiridine, lazabemide, mofegiline, salbeluzol, oxiracetam, ipidacrine, nebracetam, tacrine, velnacrine.

When the compounds include at least one asymmetric carbon atom, the products can be used in racemic mixture or in form of single enantiomer.

The active principle in the solid dispersions of the invention is in amorphous form.

By "amorphous form" of a compound it is meant a solid form of that compound that when subjected to DSC analysis does not show the melting endothermic peak.

When the active principle is in the solid dispersions of the present invention it is characterized by a higher dissolution rate and therefore a higher bioavailability than in the non dispersed form. As it will be shown in detail in the following examples, a particularly high increase in the dissolution rate occurs when the hydrophilic polymer used in the dispersion is polyvinylpyrrolidone. Thus, the use of the polyvinylpyrrolidone as the hydrophilic polymer is particularly preferred when a very fast release of the active agent is desired.

Preferably the solid dispersions of the present invention comprise one or more nitrate active principles in amounts comprised between 5% and 60% w/w and preferably between 15% and 40% w/w and the hydrophilic polymer in amount ranging from 50% to 90%, preferably between 70% and 85% w/w.

Optionally, the solid, dispersions of the present invention comprise also pharmaceuticatly acceptable excipients such as, for instance, wetting and solubilising agents in amount preferably ranging from 2% to 20%. Preferably, the solubilising agents are surfactants, and among them most preferred are polysorbates, esters and ethers of polyethylen glycols, polihydroxylated castor oil and sodium laurylsulphate. The solid dispersion of the invention can be produced by using processes known in the art such as, for instance, the methods based on co-precipitation, the methods based on melting, which consist in melting together the active agent and the carrier and then cooling the melted mass, among them it is mentioned in particular "snap-cooling" where the cooling of the melted mass is carried out on stainless steel plates, "injection molding" where the molten mass is injected into a mould, hot melt extrusion where the active principles and the carrier mixture while flowing through the extruder is contemporaneously melted, homogenized and then extruded in the form of pellets, granules and other intermediates to be used for the production of tablets (the advantage of this technique is that the mixture is subjected to high temperatures just for one minute and it is therefore suitable for active agents sensible to high temperatures), "spray congealing", where cooling of the melted mass is carried out by freezing, and the methods based on solvent evaporation, consisting in dissolving the active agent and the carrier in the same solvent, or in forming an emulsion of the active agent and of the carrier in the solvent. Among these methods a technique allowing to easily and quickly obtain solid dispersions is "spray drying". An especially preferred process for the production of the solid dispersions of the invention is a spray-drying process comprising the following steps:
a) dissolving the active principle in a solution or suspension of the hydrophilic polymer,
b) spraying the mixture obtained in step (a) through the standard nozzle of a sprayer at a flow rate ranging from 5 to 60 ml/min and at a temperature of the inlet air comprised between 50°C and 130°C.

The solution or suspension of step a) can be realized in solvents such as, for instance, water, ethanol, isopropyl alcohol, methylen chloride, butanol, cyclohexane, hexane, acetone or mixture thereof. The choice of the solvent depends on the characteristics of solubility of the active agent which has to be dissolved.

The concentration of polyvinyl pyrrolidone, hydroxypropylmethylcellulose or polyethylene glycol in said solution or suspension is comprised between 1% and 10% w/v and preferably between 2.5% and 7.5% w/v.

The active principle ingredient is added to said solution or suspension in such an amount to obtain a concentration comprised between 0.1% and 10% w/v and preferably between 0.5% and 7.5% w/v.

Optionally, at least one of the above mentioned pharmaceutically acceptable excipients can be added to the solution or suspension in such an amount as to obtain a concentration of said excipients comprised between 0.01% and 10% w/v and preferably between 0.05% and 5% w/v.

The spraying carried out in step b) is preferably carried out at a flow rate comprised between 5 and 60 ml/min and at an inlet air temperature comprised between 50°C and 130°C.

The solid dispersions of the present invention can be administrated as such, in form of powder, or used, for instance, for the production of granulates, tablets, capsules, suspensions, solutions, suppositories and aerosols.

Therefore, a further object of the present invention are pharmaceutical formulations for oral, parenteral, rectal, (trans)dermic or (trans)mucosal administration of the nitrate active principles comprising the solid dispersions of the invention.

If compared to conventional formulations, the formulations of the invention allow to improve the bioavailability and the onset of action of the nitrate active principles.

The invention will be now explained in detail by the following examples to be considered as a not limiting explanations of the invention.

### EXAMPLE 1

### Preparation of solid dispersions of the 4- acetylaminophenyl ester of the 4-nitroxybutanoic acid (NCX701).

A solution in methylene chloride/ethanol (90/10 v/v) including 0.8823% w/v of 4-acetylaminophenyl ester of the 4-nitroxybutanoic acid and 2.5% w/v of polyvinyl pyrrolidone K25 has been prepared. This has then been sprayed through the standard nozzle (inner diameter 1 mm) of a sprayer SD04 (Lab-Plant LTD, West Yorkshire, United Kingdom) at a flow rate of 20 ml/min while keeping an inlet hot air temperature of 60°C.

The obtained product has then been analysed by scanning calorimetry using a DSC T.A.2910 of T.A. INSTRUMENTS, with a heating interval and scanning rate of 10°C/min. under constant nitrogen flow. The obtained thermogram, reported in Figure 1, shows that the analysed product is amorphous: In fact no thermic event is detected in the considered temperature interval and in particular in correspondance with the melting temperature of the 4-acetylaminophenyl ester of the 4 nitroxybutanoic acid, at 78°C.

### EXAMPLE 2

### Evaluation of the dissolution rate of 4-acetylaminophenyl ester of 4 nitroxybutanoic acid in solid dispersion

The dissolution rate of the active principle of the solid dispersion produced in Example 1 has been evaluated, in comparison with the dissolution rate of the pure active principle in micronized form with the paddle method, described in F.U.X., using the following conditions:
dissolution means: distilled water
temperature: 37°C±0.5
stirring rate: 100 r.p.m.

The quantity of active ingredient released has been evaluated by UV spectrophotometry at a wavelength of 240 nm. The following table shows the average of the results obtained from three determinations, expressed as percentage of active principle dissolved at different time intervals:

| TIME (minutes) | Micronized active principle | Solid dispersion |
|---|---|---|
| 5 | 17.8 | 100 |
| 10 | 38.8 | 100 |
| 15 | 52.1 | 100 |
| 20 | 60.7 | 100 |
| 25 . | 67.5 | 100 |
| 30 | 72.4 | 100 |
| 35 | 76.4 | 100 |
| 40 | 79.7 | 100 |
| 45 | 82.6 | 100 |
| 50 | 85.2 | 100 |
| 55 | 87.3 | 100 |
| 60 | 94.9 | 100 |

As it can be observed from the table, while the active principle as such is characterized by a slow dissolution in water, when this is in the form of a solid dispersion in polyvinyl pyrrolidone its dissolution is immediate, occurring in less than five minutes.

### EXAMPLE 3

### Preparation of solid dispersions of 3-(nitroxymethyl)phenyl ester of 2-acetoxybenzoic acid (NCX4016)

Two solutions in methylene chloride/ethanol (90/10 v/v) having the following compositions have been prepared:
0.8823% w/v of 3-(nitroxymethyl)phenyl ester of the 2- acetoxybenzoic acid and 5% w/v of polyvinyl pyrrolidone K25;
2.1 % w/v of 3-(nitroxymethyl)phenyl ester of the 2- acetoxybenzoic acid and 5% w/p of polyvinyl pyrrolidone K25;
0.8823% w/v of 3-(nitroxymethyl)phenyl ester of the 2- acetoxybenzoic acid and 5% w/v of hydroxypropylmethylcellulose.

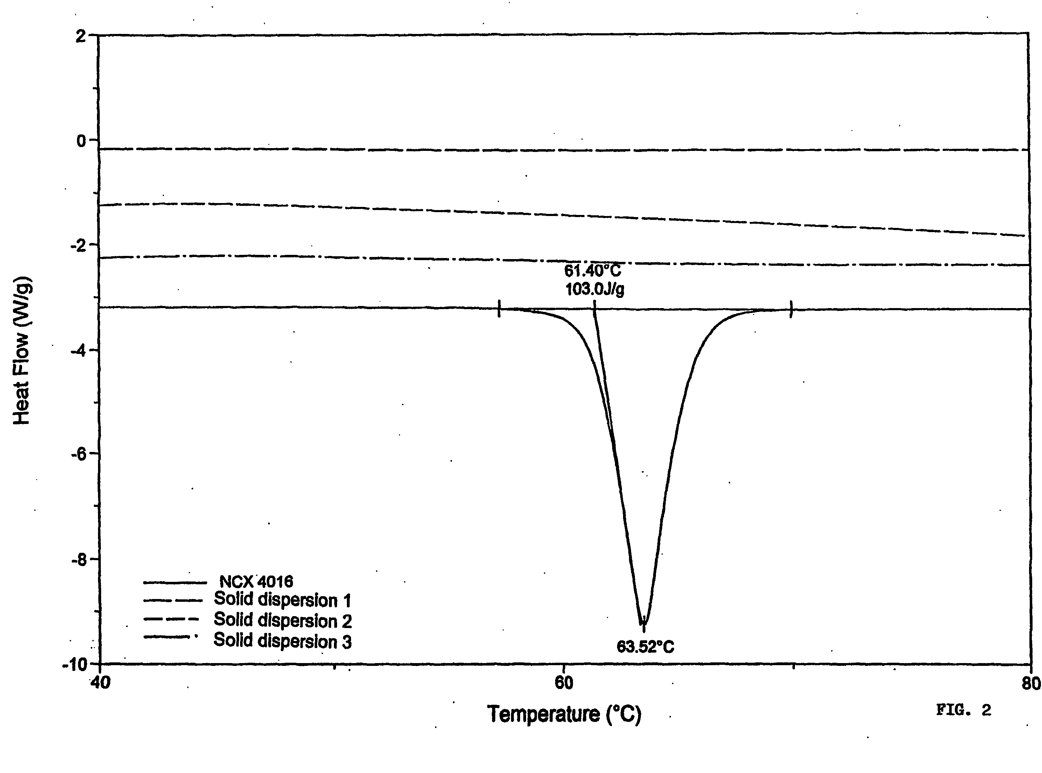

The solutions have then been sprayed as described in Example 1. The product obtained has been analysed by scanning calorimetry using a device described in the preceding example. The thermogram obtained, reported in Figure 2, shows that the analysed product is amorphous. In fact no thermic event is detected in the considered temperature interval and in particular in correspondance with the melting temperature of the 3-(nitroxymethyl)phenyl ester of 2-acetoxybenzoic acid, at 63.52°C.

### EXAMPLE 4

### Determination of the dissolution rate of NCX4016 in solid dispersion

The dissolution rate of the solid dispersion produced in the example 3 has been compared with the dissolution rate of the pure NCX4016 in micronised form using the paddle method described in F.U.X., according to the following operating conditions T = 37°C±0.5°C, stirring rate:150 rpm, dissolution means: 1% sodium lauryl sulphate solution, dissolution volume: 900 ml.

The quantity of NCX4016 released has been spectrophotometrically evaluated in continuous at a wavelength 232 nm. The following table shows the average of the results obtained from 3 determinations, expressed as percentage of active principle dissolved at different time intervals.

| TIME (minutes) | NCX4016 micronized | NCX4016 Solid dispersion 1 | NCX4016 Solid dispersion 2 | NCX4016 Solid dispersion 3 |
|---|---|---|---|---|
| 5 | 17.9 | 98.2 | 98.1 | 27.3 |
| 10 | 39.2 | 99.9 | 99.2 | 65.7 |
| 15 | 53.5 | 100 | 100 | 81.1 |
| 20 | 60.7 | 100 | 100 | 90.2 |
| 25 | 71.4 | 100 | 100 | 92.4 |
| 30 | 77 | 100 | 100 | 94.1 |
| 35 | 80.9 | 100 | 100 | 94.7 |
| 40 | 84.4 | 100 | 100 | 94.9 |
| 45 | 87 | 100 | 100 | 95.4 |
| 50 | 88.9 | 100 | 100 | 95.4 |
| 55 | 90.6 | 100 | 100 | 95.4 |
| 60 | 91.4 | 100 | 100 | 95.4 |

Also in this case, as it can be observed from the table, the dissolution rate of the active principle in all the three solid dispersions is higher than that of the active principle in pure form. Moreover, when the active principle is dispersed in polyvinyl pyrrolidone, the increase in the dissolution rate is remarkably high and an almost immediate release is observed.

### EXAMPLE 5

### Evaluation of the dissolution rate of 3-(nitroxymethyl)phenyl ester of the 2-acetoxybenzoic acid (NCX4016) in solid dispersion under condition of supersaturation

Three samples of microspheres have been exactly weighed so as to have a content of nitroaspirine of 30 mg. This quantity corresponds to about 4 times the solubility in water of the active principle.

The dissolution rate of the active principle from the above mentioned 3 solid dispersions has been compared with the dissolution rate of the pure active principle in micronized form, with the paddle method, described in F.U.X. using the following conditions:
dissolution means: distilled water
temperature: 37°c±0.5
stirring rate: 100 r.p.m.
volume = 900 ml

The quantity of NCX 4016 released has been evaluated spectrophotometrically in continuous at a wavelength 232 nm.

The samples have been taken by means of a peristaltic pump at 5 minutes intervals and for the total time of one hour.

The following table shows the average of the results obtained from three determinations, expressed as percentage of dissolved active principle at different time intervals:

| TIME (minutes) | Micronized active principle | Solid dispersion 2 |
|---|---|---|
| 5 | n.r.^{a} | 55.1 |
| 10 | n.r.^{a} | 54.3 |
| 15 | n.r.^{a} | 51.8 |
| 20 | n.r.^{a} | 48.7 |
| 25 | n.r. | 46.9 |
| 30 | Nr.^{a} | 44.7 |
| 35 | n.r.^{a} | 42.8 |
| 40 | n.r.^{a} | 40.8 |
| 45 | n.r.^{a} | 38.6 |
| 50 | n.r.^{a} | 37.3 |
| 55 | n.r.^{a} | 35.7 |
| 60 | n.r.^{a} | 34.3 |

| | | |
|---|---|---|
| a: spectrophotometrically not detectable | | |

The quantity of active agent NCX4016 dissolved after 5 minutes is about twice the solubility of the active ingredient in the dissolution means.

### EXAMPLE 6

### Preparation of solid dispersions of HCT 1026 (2-fuoro-α-methyl[1.1'biphenyl]4-acetic acid-4nitrooxy butyl ester

Two solutions in methylene chloride/ethanol (90/10 v/v) with the following compositions have been prepared:
HCT 1026 0.44% w/v; polyvinyl pyrrolidone K 30 2.5% w/v
HCT 1026 0.88% w/v; polyvinyl pyrrolidone K 30 2.5% w/v

The solutions have then been sprayed under the same conditions used in Example 1.

### EXAMPLE 7

### Evaluation of the dissolution rate of the HTC1026 in solid dispersion

The dissolution rate of the HCT 1026 from the solid dispersion 1 has been evaluated, in comparison with the dissolution rate of the pure active ingredient, with the paddle method described in F.U.X. In detail, 50 mg of the solid dispersion 1 and 7.5 mg of pure active ingredient are placed in a thermostatic container at 37°C±0.5°C in 900 ml of distilled water including 1% w/v of SDS and kept under stirring at 150 rpm. The quantity of HCT 1026 passed into the solution is continuously spectrophotometrically determined in continuous at a wavelength of 245 nm.

In the following table the average of the results obtained from three determinations is reported, expressed as percentage of active principle dissolved at different time intervals:

| TIME (minutes) | Pure HCT 1026 | Solid dispersion 1 |
|---|---|---|
| 5 | 5.84 | 81.04 |
| 10 | 16.74 | 84.74 |
| 15 | 23.6 | 85.2 |
| 20 | 29.84 | 86.13 |
| 25 | 32.78 | 85.67 |
| 30 | 37.92 | 85.85 |
| 35 | 42.57 | 86.31 |
| 40 | 47.46 | 86.68 |
| 45 | 54.94 | 86.78 |
| 50 | 58.98 | 86.78 |
| 55 | 58.98 | 87.42 |
| 60 | 64.72 | 87.79 |

The results obtained show also in this case that when the active agent is in solid dispersion in polyvinyl pyrrolidone its dissolution speed is much higher than the one of the active agent in non dispersed form, and the release of more than 80% of the active principle is observed in less than 5 minutes.

### EXAMPLE 8

### Preparation of solid dispersions of NCX 1022 (hydroxycortisone 21-[(4'-nitroxy-methyl)benzoate]

A solution of methylene chloride/ethanol (90/10 v/v) including 0.44% w/v of NCX 1022 and 2.5% w/v of polyvinyl pyrrolidone K25 has been prepared. It has then been sprayed through the standard nozzle (1 mm inner diameter) of a sprayer SD04 (Lab-Plant LTD, West Yorkshire, United Kingdom) with a flow rate of 20 ml/min. keeping a temperature of the inlet hot air of 60°C.

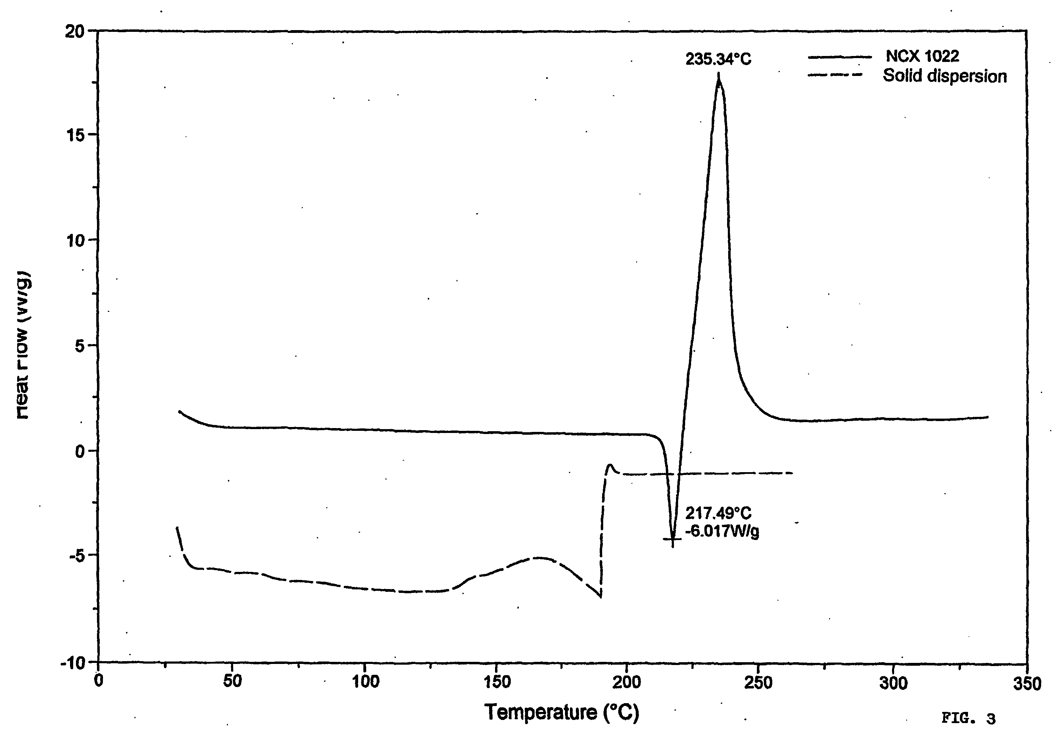

The product obtained has then been analyzed through scanning calorimetry by using the device described in the preceding examples. The thermogram obtained, reported in Figure 3, shows that the analysed product is amorphous and degrades at a temperature lower than 200°C. In fact no thermic event is detected in the considered interval of temperature and in particular in correspondance with the melting temperature of the NCX 1022.

### EXAMPLE 9

### Determination of the dissolution speed of the solid dispersion of NCX 1022

The dissolution rate of the active ingredient from the solid dispersion produced in Example 6 has been compared with the dissolution rate of the pure active ingredient, using the paddle method described in F.U.X. In detail, 40 mg of the solid dispersion or 5 mg of pure NCX 1022 have been placed in a thermostated container at 37°C±0.5°C in 500 ml of distilled water including 1% w/v of Tween 80 and kept under stirring at 100 rpm. The quantity of NCX 1022 dissolved has been spectrophotometrically determined in continuous at a wavelength of 240 nm.

The following table shows the average of the results obtained from three determinations, expressed as percentage of ingredient dissolved at different time intervals:

| TIME (minutes) | Micronized active principle | Solid dispersion |
|---|---|---|
| 5 | 4.05 | 45.56 |
| 10 | 3.91 | 49.73 |
| 15 | 3.86 | 50.36 |
| 20 | 3.81 | 49.90 |
| 25 | 3.91 | 48.84 |
| 30 | 3.77 | 47.18 |
| 35 | 3.91 | 45.60 |
| 40 | 3.77 | 43.71 |
| 45 | 4.09 | 41.93 |
| 50. | 4.33 | 40.2 |
| 55 | 4.23 | 38.82 |
| 60 | 4.32 | 37.18 |

The results obtained show that, even if the solubility of the pure active principle almost null, with a solubilisation of only 4.3% within one hour, when this is in form of a solid dispersion in polyvinyl pyrrolidone its dissolution rate and therefore its apparent solubility remarkably increase and it is possible to obtain the release of 50% of active ingredient in less than 15 minutes.

## Claims

1. Solid dispersions comprising at least one nitrate active ingredient and one hydrophilic polymer chosen among cellulose ether, polyvinyl pyrrolidone, polyethylene glycol, and wherein said active ingredient is in amorphous form.

2. Dispersions according to claim 1 wherein said polymer is polyvinyl pyrrolidone.

3. Dispersions according to claim 1 wherein said cellulose ether is hydroxypropylmethylcellulose and it has a molecular weight such that the viscosity at 20°C of a 2% solution in water is lower than 50 cps.

4. Dispersions according to claim 1 wherein polyvinyl pyrrolidone has an average molecular weight comprised between the molecular weight of polyvinyl pyrrolidone K17 and the molecular weight of polyvinyl pyrrolidone K30.

5. Dispersions according to claim 1 wherein polyethylene glycol has an average molecular weight higher than or equal to the molecular weight of polyethylene glycol 1000.

6. Dispersions according to claim 1 wherein said active ingredient is contained in amounts ranging from 10% to 50% w/w and said hydrophilic polymer is contained in amounts ranging from 50% to 90%.

7. Dispersions according to claim 6 wherein the amount of said active ingredient is between 15% and 40% w/w.

8. Dispersions according to claim 6 wherein the amount of said hydrophilic polymer is between 60% and 85%.

9. Dispersions according to claim 1 further comprising pharmaceutically acceptable excipients.

10. Dispersions according to claim 9 wherein said excipients are contained amounts comprised between 2% and 20%.

11. Dispersions according to claim 9 wherein said pharmaceutically acceptable excipients are chosen from the group consisting of wetting and solubilising agents.

12. Dispersions according to claim 11 wherein said solubilising agents are surfactants.

13. Dispersions according to claim 12 wherein said surfactants are chosen from the group comprising polysorbates, esters and ethers of polyethylene glycols, polyhydroxylated castor oil and sodiumlauryl sulphate.

14. Pharmaceutical formulations for oral, rectal, parenteral, transcutaneous, transmucosal administration of active principles comprising the solid dispersions according to claims 1 to 13.

## Patentansprüche

1. Feste Dispersionen umfassend mindestens einen Nitratwirkstoff und ein hydrophiles Polymer, ausgewählt aus Celluloseether, Polyvinylpyrrolidon, Polyethylenglycol, und worin der Wirkstoff in amorpher Form ist.

2. Dispersionen gemäß Anspruch 1, worin das Polymer Polyvinylpyrrolidon ist.

3. Dispersionen gemäß Anspruch 1, worin der celluloseether Hydroxypropylmethylcellulose ist und das Molekulargewicht derart ist, daß die Viskosität bei 20°C einer 2 % Lösung in Wasser geringer als 50 cPs ist.

4. Dispersionen gemäß Anspruch 1, worin Polyvinylpyrrolidon ein mittleres Molekulargewicht zwischen dem Molekulargewicht von Polyvinylpyrrolidon K17 und dem Molekulargewicht von Polyvinylpyrrolidon K30 aufweist.

5. Dispersionen gemäß Anspruch 1, worin Polyethylenglycol ein mittleres Molekulargewicht von mehr als oder gleich dem Molekulargewicht von Polyethylenglycol 1000 aufweist.

6. Dispersionen gemäß Anspruch 1, worin der Wirkstoff in Mengen im Bereich von 10 bis 50 % G/G enthalten ist und das hydrophile Polymer in Mengen im Bereich von 50 bis 90 % enthalten ist.

7. Dispersionen gemäß Anspruch 6, worin die Menge des Wirkstoffes zwischen 15 und 40 % G/G ist.

8. Dispersionen gemäß Anspruch 6, worin die Menge des hydrophilen Polymers zwischen 60 und 85 % ist.

9. Dispersionen gemäß Anspruch 1, die ferner pharmazeutisch annehmbare Exzipienten umfassen.

10. Dispersionen gemäß Anspruch 9, worin die Exzipienten in Mengen zwischen 2 und 20 % enthalten sind.

11. Dispersionen gemäß Anspruch 9, worin die pharmazeutisch annehmbaren Exzipienten ausgewählt sind aus der Gruppe bestehend aus Benetzungs- und Aufschlußmitteln.

12. Dispersionen gemäß Anspruch 11, worin die Aufschlußmittel Tenside sind.

13. Dispersionen gemäß Anspruch 12, worin die Tenside ausgewählt sind aus der Gruppe umfassend Polysorbate, Ester und Ether von Polyethylenglycolen, polyhydroxyliertes Rizinusöl und Natriumlaurylsulfat.

14. Pharmazeutische Formulierungen zur oralen, rektalen, parenteralen, transkutanen, transmukosalen Verabreichung von Wirkstoffen umfassend die festen Dispersionen gemäß Ansprüchen 1 bis 13.

## Revendications

1. Dispersions solides comprenant au moins un ingrédient actif nitrate et un polymère hydrophile choisi parmi un éther cellulosique, la polyvinylpyrrolidone, le polyéthylène glycol, ledit ingrédient actif étant sous forme amorphe.

2. Dispersions selon la revendication 1, dans lesquelles ledit polymère est la polyvinyl pyrrolidone.

3. Dispersions selon la revendication 1, dans lesquelles ledit éther cellulosique est l'hydroxypropylméthylcellulose et a un poids moléculaire tel que la viscosité à 20°C d'une solution à 2% dans l'eau est inférieure à 50 cps.

4. Dispersions selon la revendication 1, dans lesquelles la polyvinyl pyrrolidone a un poids moléculaire moyen compris entre le poids moléculaire de la polyvinyl pyrrolidone K17 et le poids moléculaire de la polyvinyl pyrrolidone K30.

5. Dispersions selon la revendication 1, dans lesquelles le polyéthylène glycol a un poids moléculaire moyen supérieur ou égal au poids moléculaire du polyéthylène glycol 1000.

6. Dispersions selon la revendication 1, dans lesquelles ledit ingrédient actif est présent en des quantités se situant dans la plage de 10% à 50% p/p et ledit polymère hydrophile est présent en des quantités se situant dans la plage de 50% à 90%.

7. Dispersions selon la revendication 6, dans lesquelles la quantité dudit ingrédient actif est située entre 15% et 40% p/p.

8. Dispersions selon la revendication 6, dans lesquelles la quantité dudit polymère hydrophile est comprise entre 60% et 85%.

9. Dispersions selon la revendication 1, comprenant en outre des excipients pharmaceutiquement acceptables.

10. Dispersions selon la revendication 9, dans lesquelles lesdits excipients sont présents en des quantités comprises entre 2% et 20%.

11. Dispersions selon la revendication 9, dans lesquelles lesdits excipients pharmaceutiquement acceptables sont choisis dans le groupe constitué par des agents mouillants et des agents de solubilisation.

12. Dispersions selon la revendication 11, dans lesquelles lesdits agents de solubilisation sont des surfactants.

13. Dispersions selon la revendication 12, dans lesquelles lesdits surfactants sont choisis dans le groupe comprenant les polysorbates, les esters et éthers des polyéthylène glycols, l'huile de ricin polyhydroxylée et le laurylsulfate de sodium.

14. Formulations pharmaceutiques pour une administration par voie orale, rectale, parentérale, transcutanée, transmuqueuse de principes actifs, comprenant les dispersions solides selon les revendications 1 à 13.
